# NEW EUROPEAN PATENT SPECIFICATION

(11) **EP 0 523 130 B2**
(45) Date of publication and mention of the opposition decision: **24.10.2001**
(45) Mention of the grant of the patent: 12.11.1997
(21) Application number: 91907277.7
(22) Date of filing: 25.03.1991
(51) Int. Cl.: C12N 9/96

(54) **ENZYME STABILISATION**
ENZYMSTABILISIERUNG
STABILISATION ENZYMATIQUE

(30) Priority: 24.03.1990 GB 9006642
(43) Date of publication of application: 20.01.1993
(73) Proprietor: University of Leeds Innovations Ltd., Leeds LS2 3AR (GB)
(72) Inventor: GIBSON, Timothy, David 3 Temple Avenue, Leeds LS26 0JW (GB); WOODWARD, John, Robert, Xenia, OH 45385 (US)
(74) Representative: Browne, Robin Forsythe, Dr.
(86) International application number: GB9100443
(87) International publication number: WO9114773

(56) References cited:
- EP-A- 0 074 237
- EP-A- 0 166 427
- EP-A- 0 204 045
- EP-A- 0 435 684
- FR-A- 2 182 997
- GB-A- 995 362
- GB-A- 2 090 599
- JP-A- 60 001 123
- CHEMICAL ABSTRACTS, vol. 106, no. 15, April 1987, Columbus, OH (US); p. 278, AN 115739x
- JP-LAID OPEN 60-1123 (1985)
- JP-LAID OPEN 40-10948 (1965)

## Description

This invention relates to the stabilisation of enzymes in the dry state.

PCT/GB89/01346 discloses stabilisation of proteins, particularly enzymes with combinations of cationic polyelectrolytes and cyclic polyols.

According to the present invention a method of protecting enzymes against denaturation on drying comprises the steps of mixing an aqueous solution of the enzyme with a soluble anionic polyelectrolyte and a cyclic polyol, and removing water from the solution.

Biological activity in an enzyme system, may be enhanced upon drying with the stabilisers of this invention. Freeze drying of samples may be employed. However, vacuum drying and air drying at ambient temperatures without denaturation is preferred.

In addition to retention of activity upon drying, enzymes dried in the presence of the anionic polyelectrolyte and cyclic polyol may exhibit retention of activity after prolonged storage. Although various compounds have been used to provide active dried enzyme compositions, the present invention affords excellent characteristics on prolonged storage.

The cyclic polyol may incorporate one or more alicyclic rings and may have at least one side chain. Compounds having 5 to 10 hydroxyl groups may be preferred. Non reducing polyols are preferred. Disaccharides or trisaccharides and derivatives are particularly efficacious but other cyclic polyols, eg inositol, may be used. The polyol may be chosen to suit both the enzyme or other protein and also the polyelectrolyte in question.

Use of lactitol is especially preferred although lactose, maltose, sucrose and cellobiose also may be used.

The amount of polyol may be in the preferred range 1 to 20% more preferably 2 to 10%. Percentages used in this specification are by weight to volume of aqueous solution unless indicated otherwise.

The anionic polymer is preferably a polymer with anionic groups distributed along the molecular chain. The anionic groups, which may include carboxyl, sulphonic acid, sulphate or other negatively charged ionisable groupings, may be disposed upon chain groups pendant from the chain or bonded directly to the polymer backbone.

Natural or artificial polymers may be employed. Natural polymers such as derivatised polysaccharides may be preferred since many synthetic polymers often contain residual traces of inorganic polymerisation catalysts. Alternatively synthetic polymers may be employed especially when used at very high dilution.

Carboxymethyl cellulose and sodium alginate (rich in galacturonic acid residues) are examples of carboxyl group containing polymers, dextran sulphate being an example of a sulphate containing polymer. Polymers with MW of 5,000-500,000 may be used, preferably 5.000 to 20,000 and more preferably 5,000 to 10,000. An amount of 0,05% to 10% w/v is preferred, especially 0.01 to 10%, more especially 0.5 to 2%. Use of a trace of the polyelectrolyte surprisingly affords excellent stabilisation, particularly on storage. Use of a minimal amount of the polyelectrolyte is preferred.

The pH at which the enzyme may be dried in accordance with this invention may be important to optimum retention of activity both upon drying and after subsequent storage. The optimum pH for a particular enzyme may be determined by simple experimentation. Batches of enzymes from different sources have been found to require different conditions for optimum stabilisation.

Lactate dehydrogenase has been found to retain most activity between pH 6.0 and pH 7.0, especially at pH 6.0.

Peroxidase retains most activity at pH 7.0.

Alcohol oxidase is also stabilised by an anionic polyelectrolyte/cyclic polyol combination at pH 7.0.

Drying is preferably performed at temperatures between 4°C and 50°C, especially between 25 °C to 35 °C.

The dried product may be prepared as a free running powder or it may comprise part of a test strip or other analytical or diagnostic device or apparatus.

Use of the present invention finds particular application in stabilisation of enzymes which have maximum activity at high pH, for example alkaline phosphatase. In addition the invention allows use of a particular enzyme in an assay system which employs alkaline reagents.

The present invention is now described by means of Examples but not in any limitative sense.

All stabilisation systems utilise buffers to maintain stable pH conditions.

A buffer solution containing Na₂HPO₄H₂O (10.855g) and NaH₂PO₄2H₂O (6.084g) was dissolved in 1.01 distilled water to give a solution of pH 7.0, at a concentration of 100 mM/1. This was diluted as required.

An alternative buffer is MOPS (4-morpholino propane sulphonic acid) 52.25 gm/2.5 1 distilled water to give a solution containing 100 millimoles per litre to which is added 4.0M NaOH to the required pH, eg pH 7.

Wetting agents were not used in the following examples. However, this does not preclude their use in the stabilisation systems. Byco A, a protein (gelatin) hydrolysates may be used for example as a freshly made up solution of 1% concentration.

Enzyme solutions were prepared freshly before use. Stock suspension of enzyme in ammonium sulphate solution were centrifuged and then redissolved and dialysed against a suitable buffer, or centrifuged, redissolved and used without further treatment.

Stock enzyme concentrations varied considerably, typically concentrations between 10 - 5,000 units of activity per millilitre of solution. The protein concentration being 0.05mg - 100mgcm⁻³.

The detection systems for each enzyme were those typically used in the published literature, Bergmeyer, H.U, Methods in Enzymatic Analysis, being the standard work employed.

The activity was measured kinetically at standard temperatures and the rate of reaction plotted automatically, using a Beckman Du-50 spectrophotometer fitted with a Kinetics Softpac Microprogram.

The dry preparations were produced by mixing the enzymes, buffers, anionic polyelectrolytes and polyols with stirring. Aliquots were dispersed into cuvettes and dried in a vacuum oven over silica gel as dessicant for 4 hours minimum at 30-35°C at 0.1 mM mercury.

The stability of such preparations was tested by storage at elevated temperature, 37°C or 50°C being used. Samples were stored over silica gel as dessicant, removed periodically and reconsituted in the assay buffer recommended for the enzyme determination and checked for residual activity of the enzyme.

### Example 1. Alcohol oxidase (Hansenula polymorpha. Prepared in house)

| | |
|---|---|
| Alcohol oxidase 617 units cm⁻³ (Hansenula polymorpha) | 16ul |
| Lactitol 20% | 250ul |
| Sodium alginate 2% | 250ul |
| Sodium phosphate buffer 10mM pH 7.0 | 484ul |

The mixture was mixed and 0.1 ml volumes were dried in cuvettes as described, stored at 37°C and assayed for activity in a peroxidase dye linked reaction at 505 nM.

The results are shown in Table 1.

### Example 2. L-lactate dehydrogenase (SIGMA Type II L 2500)

| | |
|---|---|
| L-Lactate dehydrogenase (dialysed), approx 4,000 U cm³ | 15ul |
| Sodium phosphate buffer pH 6.0 10mM | 1110ul |

These were mixed and added to 375ul of solutions of stabiliser as shown in Table 2.

The mixture was stirred well and 0.1 cm⁻³ placed in cuvettes which was vacuum dried as described, stored at 37°C and assayed by following the formation of Beta-NADH at 340 nm from L-lactate and Beta-NAD in glycine buffer at pH 8.9.

### Example 3. L-lactate dehydrogenase (SIGMA Type II L-250)

| | |
|---|---|
| L-lactate deydrogenase (dialysed) Approx. 4,000 U cm⁻³ | 15ul |
| Sodium phosphate buffer pH 6.0 10mM | 1110ul |

These were mixed and added to 375 ul of a solution of stabilisers as shown in Table 3.

The mixtures was stirred well and 0.1cm⁻³ placed in cuvettes, dried as described, stored at 37°C and assayed by following the formation of Beta-NADH at 340 nm from L-lactate and Beta-NAD in glycine buffer at pH 8.9.

### Example 4. Alkaline phosphatase ( Sigm p-7640 Type 1-S)

| | |
|---|---|
| Akaline phosphatase 6 ucm⁻³ (in pH 7.0 phosphate buffer 100 mM) | 30ul |
| Distilled water | 270ul |
| Sodium phosphate buffer pH 7.0 10mM | 1200ul |

These were mixed and added to 500ul of mixed solutions of stabiliser as shown in Table 4.

The mixtures were stirred well and 0.1 cm⁻³ placed in cuvettes and dried as described. The reactions were followed at 440 nM by the release of nitrophenol from the substrate 4-nitrophenol phosphate at pH 10.5 in 2 amino 2 methyl 1-propanol/HC1 buffer.

### Example 5. Horseradish peroxidase (Sigma Type II) p-8250

| | |
|---|---|
| Horseradish peroxidase 20 U cm⁻³ | 300ul |
| Sodium phosphate buffer pH 7.0 10 mM | 1200ul |

These were mixed and added to 500ul of mixed solutions of stabilisers as shown in Table 5. Experiments were also carried out using alternative peroxidases.

The mixtures were stirred well and 0.1 cm⁻³ placed in cuvettes and dried as described. Activity was followed using the colorimetric reaction of 4 aminoantipyrine and phenolsulphonic acid with hydrogen peroxide as substrate measured at 505nM.

### Example 6. Beta-Galactosidase (Sigma G-1875 Grade III)

| | |
|---|---|
| Beta-Galacosidase 1 U cm⁻³ | 25ul |
| (in 100mM pH 7.0 phosphate) | 125ul |
| Sodium phosphate bufer pH 7.0 10mM | 600ul |

These were added to 250ul of a solution of stabilisers as shown in Table 6.

The mixture was stirred well, 0.1 cm⁻³ placed in a cuvette and dried as described. The activity was measured by following the release of O-nitrophenol at 405 nM from the substrate O-nitrophenyl-Beta-D-Galactopyranoside in maleate buffer at pH 7.3.

### Example 7. Beta-Galactosidase (Sigma G-1875 Grade III)

| | |
|---|---|
| Beta-Galactosidase 1 Ucm⁻³ | 25ul |
| (in 100 mM pH 7.0 phosphate) | |
| Distilled water | 125ul |
| Sodium phosphate buffer pH 7.0 10 mM | 600ul |

These were added to 250 ul of a mixed solution of stabilisers as shown in Table 7.

The mixture was stirred well and 0.1 cm⁻³ placed in cuvettes and dried as described. The activity was followed by the same procedure as before.

### Example 8. Diacetyl reductase (Chicken liver) prepared in house

| | |
|---|---|
| Diacetyl reductase 19 units cm⁻³ | 100ul |
| Sodium phosphate buffer 10 mM pH 7.0 | |

These were added to 250ul of stabilisers as shown in Table 8.

The mixtures were stirred well and 0.1 ml volumes were dried in cuvettes as described, stored at 37°C and assayed for activity using the decrease of absorbance at 340 nM corresponding to the consuption of NADH in the reduction of diacetyl (2,3 butanedione) at pH 6.1.

### Example 9. Lactate Dehydrogenase (Sigma Type II)

The dialysed (15ul of 4000 un/cm⁻³) enzyme was added to 10mM sodium phosphate buffer 1110ul and 375ul of a solution of stabilisers was added to give the concentration shown in Table 9. The 100ul aliquots were dried as before. The activity was assayed using the same assay system as in Examples 2 and 3.

### Example 10. Maleate Dehydrogenase

20ul of a suspension of malate dehydrogenase (Sigma 410-12 from porcine heart muscle) was centrifuged at 13500 rpm. The supernatant was discarded and the precipitate dissolved in 300ul 5mM phosphate buffer pH 6.0 and dialysed for two 2-hour periods against the same buffer.

150ul of dialysed enzyme was added to 250ul sodium phosphate buffer (pH 8, 100mM) and 250ul distilled water. 350ul of stabilisers was added to the mixture to give the concentrations shown in Table 10 and the 100ul alequots were dried at 35°C.

The enzyme activity was assayed in 100mM diethanolamine buffer (pH 9.2) containing 5mM magnesium chloride and D,L-malate in excess (10 to 30mM). Beta-NAD (2.4mM) was added and the rate of production of Beta-NADH was measured by absorption at 340nm.

**Table 1**

| Preparation | Incubation 37°C | % Activity remaining relative to the activity of fresh undried enzyme |
|---|---|---|
| Alcohol oxidase (Hansenula polymorpha | zero time | 74 |
| Lactitol 5% | 1 day | 88 |
| Sodium alginate 0.5% | 12 days | 113 |
| pH 7.0 phosphate | 31 days | 147 |
| | 48 days | 118 |
| | 56 days | 100 |

Unstabilised enzyme retained 25% activity after 1 day and 4% activity after 31 days at 37°C.

**Table 2**

| Preparation | Incubation 37°C | % Activity remaining relative to the activity of fresh undried enzyme |
|---|---|---|
| L-lactate Dehydrogenase (Sigma type II) | zero time | 96 |
| Lactitol 3.5% | 1 day | 108 |
| Dextran sulphate 0.71% | 13 days | 96 |
| pH 6.0 phosphate | 21 days | 106 |
| | 30 days | 93 |
| | 54 days | 88 |

Unstabilised enzyme retained 55% activity after 1 day and 46% activity after 54 days at 37°C

**Table 3**

| Preparation | Incubation 37°C | % Activity remaining relative to the activity of fresh undried enzyme |
|---|---|---|
| L-lactate dehydrogenase (Sigma type II) | zero time | |
| Lactitol 2.75% | 1 day | 95 |
| Sodium Alginate 0.22% | | |
| pH 6.0 phosphate | 13 days | 89 |

Unstabilised enzyme retained 55% activity after 1 day and 46% activity after 54 days at 37°C.

**Table 4**

| Preparation | | Incubation 37°C | % Activity remaining relative to the density of final undried enzyme |
|---|---|---|---|
| Alkaline phosphase (Sigma type 1-5) | | zero time | 100 |
| pH 7.0 phosphate | | | |
| a. | Lactitol 3.5% | 1 day | 89 |
| | Dextran sulphate 0.7% | 7 days | 86 |
| b. | Lactitol 5% | zero time | 83 |
| | Dextran sulphate 0.71% | 15 days | 95 |
| c. | Lactitol 2.7% | zero time | 100 |
| | Carboxymethyl cellulose | 15 days | 92 |
| | 0.56% | | |

Unstabilised enzyme retained 80% activity after 1 day and 38% activity after 23 days at 37°C.

**Table 5**

| Preparation | | Incubation 50°C | % Activity remaining relative to the density of fresh undried enzyme |
|---|---|---|---|
| Horseradish peroxidase | | zero time | 99 |
| (Sigma type II) P8250 | | | |
| a. | Lactitol 2.75% | 1 day | 69 |
| | Sodium | 7 days | 79 |
| | carboxymethylcellulose | | |
| | 0.55% | | |
| | pH 7.0 phosphate 10mM | 15 days | 79 |
| | | 23 days | 74 |
| b. | Lactitol 2.7% | zero time | 102 |
| | Sodium | 15 days | 73 |
| | carboxymethylcellulose | | |
| | 0.56% | | |
| c. | Lactitol 2.7% | zero time | 102 |
| | Dextran sulphate 0.71% | 15 days | 73 |
| Horseradish peroxidase | | zero time | 71 |
| (Biozyme HRP-4b) | | | |
| a. | Lactitol 5% Dextran sulphate | 17 days | 56 |
| | 1% | | |
| b. | Lactitol Carboxymethyl | zero time | 80 |
| | cellulose | | |
| | | 17 days | 35 |
| Horseradish peroxidase | | | |
| (Biozyme HRP-5, 90% Isoenzyme C) | | | |
| a. | Lactitol 5% | zero time | 87 |
| | Dextran sulphate 1% | 17 days | 60 |
| b. | Lactitol 5% | zero time | 78 |
| | Carboxymethylcellulose 1% | 17 days | 65 |

Unstabilised enzyme retained 62% ativity after 1 day and 21% activity after 15 days at 50°C.

**Table 6**

| Preparation | Incubation 50°C | % Activity remaining relative to the activity of fresh undried enzyme |
|---|---|---|
| Beta-Galacosidase (Sigma) | zero time | 109 |
| Lactitol 3.5% | 1 day | 91 |
| Dextran sulphate 0.71% | 7 days | 86 |
| pH 7.0 phosphate | 10 days | 87 |
| | 36 days | 81 |

Unstabilised enzyme retained 66% activity after 7 days and 52% activity after 36 days at 50°C.

**Table 7**

| Preparation | Incubation 50°C | % Activity remaining relative to the activity of fresh undried enzyme |
|---|---|---|
| Beta-Galactosidase | zero time | 114 |
| (Sigma Type) | | |
| Lactitol 2.75% | | |
| Sodium carboxymethyl cellulose | 1 day | 91 |
| pH 7.0 phosphate 10mM | 7 days | 77 |
| | 10 days | 89 |
| | 36 days | 72 |

Unstabilised enzyme retained 66% activity after 7 days and 52% activity after 36 days at 50°C.

**Table 8**

| Preparation | | Incubation 37°C | % Activity remaining relative to the activity of fresh undried enzyme |
|---|---|---|---|
| Diacetyl reductase (Chicken | | zero time | 216 |
| liver) | | | |
| pH 7.0 phosphate 10mM | | | |
| a. | Lactitol 5% | 8 days | 144 |
| | Dextran sulphate 1% | 21 days | 134 |
| b. | Lactitol 5% | zero time | 64 |
| | Carboxymethyl cellulose 1% | 8 days | 36 |
| c. | Lactitol 5% | zero time | 68 |
| | Sodium alginate 0.5% | 8 days | 60 |

Unstabilised enzyme retained 9% activity ater 8 days at 37°C.

**Table 9**

| Lactate dehydrogenase (Sigma Type II) | | |
|---|---|---|
| Lactitol 5% | zero time | 96 |
| Dextran sulphate 0.7% | 54 days | 88 |

Unstabilised enzume retained 55% activity after 1 day and 46% activity after 54 days at 37°C.

**Table 10**

| Maleate dehydrogenase (Sigma) | | |
|---|---|---|
| Lactitol 5% | zero time | 77 |
| Carboxymethylcellulose 1% | 20 days | 89 |

Unstabilised enzyme retained 14% activity on drying, (aero time) and 2% activity after 20 days at 37°C

## Claims

1. A method of protecting an enzyme against denaturation on drying comprising the steps of:
mixing an aqueous solution of the enzyme with a soluble anionic polyelectrolyte, a cylic polyol, and
removing water from the solution.

2. A method as claimed in claim 1, wherein said polyelectrolyte comprises an anionic functionalised polysaccharide.

3. A method as claimed in claim 2, wherein the polyelectrolyte is selected from: dextran sulphate, sodium aliginate or carboxymethylcellulose.

4. A method as claimed in any preceding claim wherein the polyol is selected from: di- or trisaccharides.

5. A method as claimed in claim 4, wherein the polyol is selected from lactitol, lactose, maltose, sucrose and cellobiose.

6. A method as claimed in any preceding claim, wherein water is removed at a temperature of between 4° and 50°C.

7. A method as claimed in claim 6, wherein the temperature is 25 ° to 35 °C.

8. A method as claimed in any preceding claim, wherein the amount of anionic polyelectrolyte in the aqueous solution is from 0.005 to 10% w/v.

9. A method as claimed in claim 8, wherein the amount is 0.01 to 10%.

10. A method as claimed in claim 9, wherein the amount is 0.5 to 2%.

11. A method of protecting an enzyme against denaturation on storage comprising use of a method as claimed in any preceding claim.

12. A dried enzyme preparation protected against denaturation on storage, consisting of the enzyme, a soluble anionic polyelectrolyte, a cyclic polyol, a buffer and optionally a wetting agent.

## Revendications

1. Procédé pour protéger une enzyme contre sa dénaturation susceptible d'intervenir au cours de son séchage, comprenant les étapes consistant à :
mélanger une solution aqueuse de l'enzyme avec un polyélectrolyte anionique soluble, un polyol cyclique, et
éliminer l'eau de la solution.

2. Procédé selon la revendication 1, selon lequel ledit polyélectrolyte comprend un polysaccharide anionique fonctionnalisé.

3. Procédé selon la revendication 2, selon lequel ledit polyélectrolyte est choisi parmi le sulfate de dextran, l'alginate de sodium et la carboxyméthylcellulose.

4. Procédé selon l'une quelconque des revendications précédentes, selon lequel le polyol est choisi parmi les di- et tri-saccharides.

5. Procédé selon la revendication 4, selon lequel le polyol est choisi parmi le lactitol, le lactose, le maltose, le saccharose et le cellobiose.

6. Procédé selon l'une quelconque des revendications précédentes, selon lequel l'eau est retirée à une température comprise entre 4 et 50°C.

7. Procédé selon la revendication 6, selon lequel la température est comprise entre 25 et 35°C.

8. Procédé selon l'une quelconque des revendications précédentes, selon lequel la proportion du polyélectrolyte anionique dans la solution aqueuse est comprise entre 0,005 et 10 % (poids/volume).

9. Procédé selon la revendication 8, selon lequel la proportion est comprise entre 0,01 et 10 %.

10. Procédé selon la revendication 9, selon lequel la proportion est comprise entre 0,5 et 2 %.

11. Procédé pour protéger une enzyme contre sa dénaturation susceptible d'intervenir au cours de son stockage, comprenant l'utilisation d'un procédé selon l'une quelconque des revendications précédentes.

12. Préparation enzymatique séchée protégée de sa dénaturation au cours de son stockage consistant en une enzyme, un polyélectrolyte anionique soluble, un polyol cyclique, un tampon et éventuellement un agent mouillant.

## Patentansprüche

1. Verfahren zum Schützen eines Enzyms gegen Denaturierung beim Trocknen, welches die Schritte umfaßt:
Mischen einer wässrigen Lösung des Enzyms mit löslichem, anionischen Polyelektrolyt, cyclischem Polyol und Entfernen von Wasser aus der Lösung.

2. Verfahren nach Anspruch 1, wobei der Polyelektrolyt anionisch funktionalisiertes Polysaccharid umfaßt.

3. Verfahren nach Anspruch 2, wobei der Polyelektrolyt aus Dextransulfat, Natriumalginat oder Carboxycellulose ausgewählt wird.

4. Verfahren nach einem der vorhergehenden Ansprüche, wobei das Polyol aus Di- oder Trisacchariden ausgewählt wird.

5. Verfahren nach Anspruch 4, wobei das Polyol aus Lactit, Lactose, Maltose, Saccharose und Cellobiose ausgewählt wird.

6. Verfahren nach einem der vorhergehenden Ansprüche, wobei Wasser bei einer Temperatur zwischen 4°C und 50°C entfernt wird.

7. Verfahren nach Anspruch 6, wobei die Temperatur 25°C bis 35°C beträgt.

8. Verfahren nach einem der vorhergehenden Ansprüche, wobei die Menge an anionischem Polyelektrolyt in der wässrigen Lösung 0,005 bis 10 % (Gew./Vol.) beträgt.

9. Verfahren nach Anspruch 8, wobei die Menge 0,01 bis 10 % beträgt.

10. Verfahren nach Anspruch 9, wobei die Menge 0,5 bis 2 % beträgt.

11. Verfahren zum Schützen eines Enzyms gegen Denaturierung bei Lagerung, welches die Verwendung eines Verfahrens, wie in einem der vorhergehenden Ansprüche beansprucht, umfaßt.

12. Getrocknete Enzymzubereitung, die gegen Denaturierung bei Lagerung geschützt ist, die aus dem Enzym, löslichem anionischen Polyelektrolyt, cyclischem Polyol, Puffer und wahlweise einem Benetzungsmittel besteht.
